# EUROPEAN PATENT APPLICATION

(11) **EP 4 802 998 A2**
(43) Date of publication of application: **09.09.2026**
(21) Application number: 26162296.3
(22) Date of filing: 04.03.2026
(51) Int. Cl.: A61B 5/145, A61B 5/1486

(54) **CONTINUOUS GLUCOSE CONCENTRATION MONITORING METHOD BASED ON A MULTI-WORKING-ELECTRODE SYSTEM, CONTINUOUS GLUCOSE CONCENTRATION MONITORING DEVICE BASED ON A MULTI-WORKING-ELECTRODE SYSTEM, COMPUTER PROGRAM, COMPUTER-READABLE MEDIUM AND METHOD FOR PREPARING CONTINUOUS GLUCOSE CONCENTRATION MONITORING DEVICE BASED ON A MULTI-WORKING-ELECTRODE SYSTEM**

(30) Priority: 07.03.2025 CN 202510272581; 07.03.2025 CN 202510272969
(71) Applicant: Jiangsu Yuwell POCTech Biotechnology Co., Ltd., Zhenjiang, Jiangsu 212300 (CN); Jiangsu Yuekai Biotechnology Co., Ltd., Nanjing, Jiangsu 210018 (CN); Zhejiang Poctech Co., Ltd., Huzhou, Zhejiang 313001 (CN)
(72) Inventor: ZHANG, Yanan, Nanjing, 210018 (CN); WU, Shuyun, Nanjing, 210018 (CN); WANG, Jie, Nanjing, 210018 (CN); FANG, Guoqing, Nanjing, 210018 (CN); ZHOU, Xuetong, Nanjing, 210018 (CN)
(74) Representative: Lorenz Seidler Gossel Part. mbB

(57) **Abstract**

This application discloses a continuous glucose concentration monitoring method based on a multi-working-electrode system, a continuous glucose concentration monitoring device based on a multi-working-electrode system, a computer program, a computer-readable medium and a method for preparing continuous glucose concentration monitoring device based on a multi-working-electrode system. The method includes: obtaining, by using a first working electrode, a first current value used for representing a glucose concentration of a host, and obtaining, by using a second working electrode, a second current value used for representing the glucose concentration of the host, where the first working electrode and the second working electrode are separately and independently disposed on a same substrate, and the first working electrode includes a first parameter, and the second working electrode includes a second parameter; and determining a target glucose concentration according to the first current value as well as the first parameter and/or the second parameter as well as the second current value, where the first parameter is determined based on the second parameter and the second current value. In this application, a measurement error caused by a single electrode is reduced by using the first working electrode and the second working electrode, to improve accuracy of glucose concentration monitoring, and reduce a monitoring result deviation caused by an electrode difference.

## Description

### TECHNICAL FIELD

This application relates to the field of blood glucose measurement technologies, and in particular, to a continuous glucose concentration monitoring method based on a multi-working-electrode system, a continuous glucose concentration monitoring device based on a multi-working-electrode system, a computer program, a computer-readable medium and a method for preparing continuous glucose concentration monitoring device based on a multi-working-electrode system.

### BACKGROUND

With an increase in the number of persons suffering from diabetes, real-time monitoring of blood glucose concentrations becomes especially important. An existing blood glucose monitoring method mainly includes fingertip blood detection and a Continuous Glucose Monitoring (CGM) system. Although an operation of the fingertip blood detection is simple, blood samples need to be frequently collected, causing inconvenience to a patient. The CGM system continuously monitors blood glucose concentrations, and can provide more comprehensive blood glucose information. An existing Continuous Glucose Monitoring (CGM) system can provide real-time and continuous blood glucose information, and therefore, is increasingly widely applied to diabetes management.

In an existing CGM technology, a single working electrode is usually used to monitor a glucose concentration. However, because manufacturing processes and use conditions of different working electrodes are different, effects of a single electrode on monitoring of a glucose concentration are also different, leading to an inaccurate monitoring result. Use of a plurality of working electrodes can improve accuracy of a monitoring result to some extent. However, different working electrodes have different manufacturing processes and use conditions, and it is difficult for a current manufacturing method to satisfy a requirement of a multi-working-electrode system. Therefore, it is urgently necessary to search for a new manufacturing method.

### SUMMARY

Embodiments of this application provide a continuous glucose concentration monitoring method based on a multi-working-electrode system, a continuous glucose concentration monitoring device based on a multi-working-electrode system, a computer program, a computer-readable medium and a method for preparing continuous glucose concentration monitoring device based on a multi-working-electrode system, to resolve the technical problem put forward in the background.

A first aspect of the embodiments of this application provides a continuous glucose concentration monitoring method based on a multi-working-electrode system. The method includes:
obtaining, by using a first working electrode, a first current value used for representing a glucose concentration of a host, and obtaining, by using a second working electrode, a second current value used for representing the glucose concentration of the host, where the first working electrode and the second working electrode are separately and independently disposed on a same substrate; and the first working electrode includes a first parameter, and the second working electrode includes a second parameter; and
determining a target glucose concentration according to the first current value as well as the first parameter and/or the second parameter as well as the second current value,
where the first parameter is determined based on the second parameter and the second current value.
Through the foregoing content, the embodiments of this application have the following beneficial effects:
   The current values used for representing the glucose concentration of the host are independently obtained by using both the first working electrode and the second working electrode, so that a monitoring result error caused by a single electrode is reduced, and the accuracy of monitoring the glucose concentration can be improved. Moreover, because the first parameter of the first working electrode is set to be determined based on the second parameter of the second working electrode, only the second parameter needs to be determined before monitoring, thereby reducing difficulty in determining the first parameter and improving precision of determining the first parameter. In addition, such interdependent parameter setting can optimize performance of the electrodes, so that the performance of the electrodes can better meet an actual monitoring requirement. In addition, the multi-working-electrode system provides a redundant monitoring mechanism, and when one electrode has performance degradation or breaks down, another electrode continues to provide reliable monitoring data, thereby improving stability and reliability of the entire system.

Further, the outputting a target glucose concentration according to the first current value as well as the first parameter and/or the second parameter as well as the second current value includes:
obtaining a first glucose concentration corresponding to the first working electrode based on the first current and the first parameter;
obtaining a second glucose concentration corresponding to the second working electrode based on the second current value and the second parameter; and
outputting the target glucose concentration based on the first glucose concentration and/or the second glucose concentration.

Through the foregoing content, the embodiments of this application have the following beneficial effects:
Because each electrode responds to different interference factors differently, environmental interference and cross-sensitivity are identified and eliminated more effectively by comparing measurement results of two electrode, thereby improving reliability of a monitoring result.

Further, the determining the first parameter based on the second parameter and the second current value includes:
obtaining a second glucose concentration corresponding to the second working electrode based on the second current value and the second parameter, when the glucose concentration of the host falls within a first threshold range, obtaining a second glucose concentration corresponding to the second working electrode based on the second current value and the second parameter; and
obtaining the first parameter based on the glucose concentration corresponding to the second working electrode and the first current value.

Through the foregoing content, the embodiments of this application have the following beneficial effects:
When the glucose concentrations fall within the first threshold range, the glucose concentration outputted by using the first working electrode and the glucose concentration outputted by using the second working electrode are substantially the same. Therefore, when the glucose concentration of the host falls within the first threshold range, the glucose concentration corresponding to the second working electrode is obtained according to the second current value and the second parameter, and the first parameter of the first working electrode is calibrated according to the glucose concentration corresponding to the second working electrode and the first current value, thereby improving the accuracy of the measurement result of the first working electrode.

Further, the outputting the target glucose concentration based on the first glucose concentration and/or the second glucose concentration includes:
outputting the first glucose concentration or the second glucose concentration as the target glucose concentration when the second glucose concentration is between a first preset value and a second preset value (a normal glucose concentration), where the first preset value is less than the second preset value.

Through the foregoing content, the embodiments of this application have the following beneficial effects:
When the second glucose concentration is between the first preset value and the second preset value, both the first working electrode and the second working electrode can provide reliable measurement data, and the system flexibly selects the first glucose concentration or the second glucose concentration as the target glucose concentration.

Further, when the second glucose concentration is less than the first preset value, the method further includes:
outputting the first glucose concentration as the target glucose concentration.

Through the foregoing content, the embodiments of this application have the following beneficial effects:
When the second glucose concentration is less than the first preset value (a low glucose concentration), the second working electrode causes interference due to an oxygen effect. This interference can be avoided by using the data of the first working electrode as the target glucose concentration, thereby improving measurement accuracy.

Further, when the second glucose concentration is greater than the second preset value, the method further includes:
determining a first weight value of the first working electrode and a second weight value of the second working electrode based on the first parameter and the second parameter respectively; and
determining the target glucose concentration based on the first weight value, the second weight value, the first glucose concentration, and the first glucose concentration.

Through the foregoing content, the embodiments of this application have the following beneficial effects:
When the second glucose concentration is greater than the second preset value (a high glucose concentration), the first working electrode is affected due to diffusion of hydrogen oxide, and the second working electrode is affected by the oxygen effect. Therefore, a measurement result is optimized according to a weight ratio of the two electrodes, to reduce a measurement error caused by a difference between characteristics of the electrodes.

Further, the first parameter includes a first sensitivity and a background current; the second parameter includes a second sensitivity; and a method for determining the first weight value and the second weight value includes:
separately determining the first weight value of the first working electrode and the second weight value of the second working electrode based on a ratio of the first sensitivity to the second sensitivity, where a product of the first sensitivity and the first weight value is equal to a product of the second sensitivity and the second weight value.

Through the foregoing content, the embodiments of this application have the following beneficial effects:
By ensuring that the product of the first sensitivity and the first weight value is equal to the product of the second sensitivity and the second weight value, it is ensured that the weighted sensitivities of the two electrodes keep the same, thereby improving the accuracy of the measurement result.

The outputting the target glucose concentration based on the first glucose concentration and/or the second glucose concentration includes:
using the second glucose concentration as the target glucose concentration if a difference between the first glucose concentration and the second glucose concentration exceeds a third preset value and/or a difference between the first current value and the second current value exceeds a fourth preset value.

If the difference between the first glucose concentration and the second glucose concentration exceeds the first preset value and/or the difference between the first current value and the second current value exceeds the second preset value, it is determined that oxygen in an interstitial liquid near an electrode is insufficient, and the insufficient oxygen causes incomplete reaction of the electrode, thereby affecting the current value. The second glucose concentration of the second working electrode is used as a target value, to reduce this error. In addition, in this embodiment of this application, working can continue in an environment with insufficient oxygen, thereby improving adaptability and reliability of the system.

Further, the method further includes:
setting, if a change rate of the glucose concentration corresponding to the first working electrode and/or the second working electrode exceeds a fifth preset value, the corresponding working electrode to be failed; or
setting, if a change rate of the glucose concentration corresponding to the first working electrode and/or the second working electrode is less than a sixth preset value, the corresponding working electrode to be failed, where the sixth preset value is less than the fifth preset value.

Through the foregoing content, the embodiments of this application have the following beneficial effects:
By monitoring the change rate of the glucose concentration, a working status of an electrode is determined in real time, thereby ensuring continuity and stability of a measurement process. In addition, a failed electrode causes an increase in a measurement error. The failed electrode is identified and excluded in advance, so that the measurement error is reduced, and accuracy of data is ensured.

Further, the outputting the target glucose concentration based on the first glucose concentration and/or the second glucose concentration includes:
obtaining a first temperature coefficient of the first working electrode and a second temperature coefficient of the second working electrode;
determining a temperature difference based on the first temperature coefficient, the second temperature coefficient, the first glucose concentration, and the second glucose concentration, where the temperature difference is a difference between a current actual measured temperature and a preset temperature; and
outputting the target glucose concentration based on the temperature difference, the first temperature coefficient, and the first glucose concentration; or
outputting the target glucose concentration based on the temperature difference, the second temperature coefficient, and the second glucose concentration.

Through the foregoing content, the embodiments of this application have the following beneficial effects:
A temperature is one of important factors affecting a response of an electrode. By considering a temperature coefficient, the system can compensate for a blood glucose concentration measurement error caused by a temperature change, thereby improving accuracy of a measurement result. In addition, the temperature difference is determined and the temperature coefficient is applied, so that impact of the temperature on the measurement result is reduced, and the measurement accuracy of the blood glucose concentration is improved.

A second aspect of one or more embodiments of this application provides a continuous glucose concentration monitoring apparatus based on a multi-working-electrode system. The apparatus includes:
a data obtaining module, configured to obtain, by using a first working electrode, a first current value used for representing a glucose concentration of a host, and obtain, by using a second working electrode, a second current value used for representing the glucose concentration of the host, where the first working electrode and the second working electrode are separately and independently disposed on a same substrate, and a first parameter of the first working electrode is determined based on a second parameter of the second working electrode; and
a data processing module, configured to output a target glucose concentration according to the first current value, the first parameter, and the second current value, where the first parameter is determined based on the second parameter.

This application provides a continuous glucose concentration monitoring device based on a multi-working-electrode system, comprising: a substrate, a first working electrode, wherein the first working electrode has a first parameter, a second working electrode, wherein the second working electrode has a second parameter; wherein the first working electrode and the second working electrode are independently disposed on the substrate, and a processing unit configured to perform the steps of the method according to the foregoing first aspect.

A third aspect of one or more embodiments of this application provides A computer device, including a memory, a processor, and a computer program stored in the memory and executable on the processor, where the processor, when executing the computer program, implements the method according to the foregoing first aspect.

A fourth aspect of one or more embodiments of this application provides a computer-readable storage medium. The computer-readable storage medium stores a computer program, where the computer program, when executed by a processor, implements the method according to the foregoing first aspect.

A fifth aspect of one or more embodiments of this application provides a computer program product. The computer program product, when run on a computer device, causes the computer device to perform the method according to the foregoing first aspect.

At least one of the above technical solutions employed in the embodiments of this application can achieve the following beneficial effects.

In this application, the current values used for representing the glucose concentration of the host are independently obtained by using both the first working electrode and the second working electrode, so that a monitoring result error caused by a single electrode is reduced, and the accuracy of monitoring the glucose concentration can be improved. Moreover, because the first parameter of the first working electrode is set to be determined based on the second parameter of the second working electrode, only the second parameter needs to be determined before monitoring, thereby reducing difficulty in determining the first parameter and improving precision of determining the first parameter. In addition, such interdependent parameter setting can optimize performance of the electrodes, so that the performance of the electrodes can better meet an actual monitoring requirement. In addition, the multi-working-electrode system provides a redundant monitoring mechanism, and when one electrode has performance degradation or breaks down, another electrode continues to provide reliable monitoring data, thereby improving stability and reliability of the entire system.

This application provides a method for preparing a multi-working-electrode system for detecting a glucose concentration of a host, including:
disposing a first working electrode and a second working electrode at an interval on a substrate, where
the first working electrode is configured to independently generate a first current value used for representing a first physiological parameter, the second working electrode is configured to independently generate a second current value used for representing a second physiological parameter, at least one of the first physiological parameter and the second physiological parameter is a glucose concentration, and an output glucose concentration of the host is obtained based on the first current value and the second current value.

Optionally, the first physiological parameter and the second physiological parameter are both glucose concentrations, the first working electrode includes a glucose oxidase, and the second working electrode includes a glucose dehydrogenase.

In this solution, both the first physiological parameter and the second physiological parameter are glucose concentrations, and the corresponding current values of the first working electrode and the second working electrode may be cross-referenced to determine whether the current value representing the glucose concentration of the host is accurate, thereby improving the accuracy of glucose concentration monitoring. The first working electrode includes a glucose oxidase, which can have relatively high monitoring accuracy in a low blood glucose environment. The second working electrode includes a glucose dehydrogenase, which can have relatively high monitoring accuracy in a high blood glucose environment.

Optionally, the first working electrode comprises a first outer membrane wrapping at least the first working electrode and the second working electrode comprises a second outer membrane wrapping at least the second working electrode; and optionally an oxygen permeability of the first outer membrane is greater than an oxygen permeability of the second outer membrane.

Optionally, the substrate includes a first substrate portion and a second substrate portion that are disposed independently of each other, and the disposing a first working electrode and a second working electrode at an interval on a substrate includes:
disposing the first working electrode on the first substrate portion, and forming a first outer membrane wrapping at least the first working electrode;
disposing the second working electrode on the second substrate portion, and forming a second outer membrane wrapping at least the second working electrode; and
combining the first substrate portion and the second substrate portion into the substrate.

In the foregoing preparation method, working parts of the first working electrode and the second working electrode are separately prepared. The first working electrode and the second working electrode are manufactured on the first substrate portion and the second substrate portion respectively without interfering with each other and are independent of each other. In this way, the first working electrode and the second working electrode can both play a detection role in respective optimized conditions. The substrate is formed by means of assembly, the two working electrodes may be integrated with a reference electrode and a counter electrode on the substrate. In addition, in this manner, locations of the first working electrode and the second working electrode can be close to each other, and measurement implantation areas and environments are substantially the same.

Optionally, the first working electrode and the second working electrode are disposed on the same substrate and are disposed at an interval in a longitudinal direction of the substrate, and the method further includes:
forming a first outer membrane wrapping at least the first working electrode on the substrate; and
forming a second outer membrane wrapping at least the second working electrode on the substrate, where the first outer membrane and the second outer membrane are disposed at an interval in the longitudinal direction of the substrate.

In this manner, the first working electrode and the second working electrode are disposed on the same substrate, so that an assembly process can be simplified, and a working part of the first working electrode and a working part of the second working electrode are separately prepared subsequently.

Optionally, the method further includes:
the substrate includes a first part and a second part that are at least partially connected to each other, bending the first part and the second part in a direction away from each other to partially separate the two parts, where the first working electrode is disposed at the first part, and the second working electrode is disposed at the second part;
forming a first outer membrane wrapping at least the first working electrode on the first part, and forming a second outer membrane wrapping at least the second working electrode on the second part; and
restoring the first part and the second part to a non-separated state.

This solution is used as a first solution of preparing the first working electrode and the second working electrode on the same substrate. To prepare the working parts of the two working electrodes, the first part and the second part that are at least partially connected on the substrate are bent in a direction away from each other, to partially separate the two parts. Then, an operation of wrapping the first working electrode with the first outer membrane and wrapping the second working electrode with the second outer membrane is performed. After the operation is completed, the first part and the second part are restored to the substrate in the non-separated state, thereby completing the preparation of the dual-working-electrode system. In this solution, the first working electrode and the second working electrode are disposed at an interval, and the first outer membrane and the second outer membrane are disposed at an interval, so that working independence of the first working electrode and the second working electrode can be maintained, thereby improving detection accuracy.

Optionally, the method further includes:
forming a first outer membrane wrapping at least the first working electrode and the second working electrode on the substrate; and
covering the first outer membrane with a second outer membrane wrapping the second working electrode.

This solution is used as a second solution of preparing the first working electrode and the second working electrode on the same substrate. In this solution, the first working electrode and the second working electrode are not separated. Both the first working electrode and the second working electrode is coated with the first outer membrane. The two working electrodes are both covered, and then the first outer membrane is coated with the second outer membrane wrapping the second working electrode. A preparation process of this solution is simpler, and does not interfere with respective working of the two working electrodes.

Optionally, the method further includes:
forming a second outer membrane wrapping at least the first working electrode and the second working electrode on the substrate.

This solution is used as a third solution of preparing the first working electrode and the second working electrode on the same substrate. In this manner, the first working electrode and the second working electrode are wrapped with only the second outer membrane. This solution is the simplest. The first working electrode in this application can only work with a low blood glucose concentration. Therefore, when the first working electrode is wrapped with the second outer membrane, as long as a small amount of oxygen penetrates to the first working electrode for reaction, measurement can be performed. Therefore, even if the first working electrode is wrapped with the second outer membrane, a test requirement of the first working electrode can still be satisfied. Measurement of the first working electrode in this section of area can replace measurement of the second working electrode, thereby avoiding a case of an oxygen effect of the second working electrode in this section of area.

Optionally, an oxygen permeability of the first outer membrane is greater than an oxygen permeability of the second outer membrane.

In this solution, by setting a relationship between the oxygen permeability of the first outer membrane and the oxygen permeability of the second outer membrane, it is ensured that the first working electrode and the second working electrode can function in respective corresponding working environments, thereby improving the accuracy of glucose concentration monitoring.

Optionally, the method further includes:
forming a first wire and a second wire on the substrate, where the first wire is connected to a first contact and the first working electrode on the substrate, the second wire is connected to a second contact and the second working electrode on the substrate, and the first wire and the second wire are insulated from each other.

In this solution, by insulating the first wire and the second wire from each other, it is ensured that the first working electrode and the second working electrode can independently work, and do not interfere with each other.

This application provides a multi-working-electrode system for detecting a glucose concentration of a host, including:
a substrate;
a first working electrode, including a glucose oxidase; and
a second working electrode, including a glucose dehydrogenase, where
the first working electrode and the second working electrode are disposed at an interval on the substrate, independently generate a first current value and a second current value used for representing a glucose concentration of the host respectively, and determine the glucose concentration of the host according to the first current value and the second current value; and
the first working electrode is wrapped with a first outer membrane, the second working electrode is wrapped with a second outer membrane, and an oxygen permeability of the first outer membrane is greater than an oxygen permeability of the second outer membrane.

According to the method for preparing a multi-working-electrode system provided in this application, the first working electrode is configured to independently generate the first current value used for representing the first physiological parameter, the second working electrode is configured to independently generate the second current value used for representing the second physiological parameter, and at least one of the first physiological parameter and the second physiological parameter is set to a glucose concentration. In this way, a plurality of working electrodes can adapt to each other in the same system. When a working electrode obtains a current value representing a glucose concentration of a host, a current value corresponding to a physiological parameter of another working electrode can be used as a reference object, to determine whether the current value representing the glucose concentration of the host is accurate. In this way, a more accurate output glucose concentration is obtained based on the current values corresponding to the two working electrodes, so that a measurement error caused by a single electrode can be avoided, and accuracy of glucose concentration monitoring can be improved. In addition, the another working electrode is configured to detect a physiological parameter of the host other than the glucose concentration, so that the applicable range of the multi-working-electrode system can be expanded and practicality can be improved.

### BRIEF DESCRIPTION OF THE DRAWINGS

To describe technical solutions in embodiments of this application or in the prior art more clearly, the following briefly describes accompanying drawings required for describing the embodiments or the prior art. Apparently, the accompanying drawings in the following description show merely some embodiments in this application, and a person of ordinary skill in the art derives other drawings from these accompanying drawings without creative efforts. In the accompanying drawings:
FIG. 1 is a schematic diagram of a measurement signal of a first working electrode according to an embodiment of this application;
FIG. 2 is a schematic diagram of a measurement signal of a second working electrode according to an embodiment of this application;
FIG. 3 is a schematic diagram of calibration according to an embodiment of this application;
FIG. 4 is a schematic diagram of signal comparison according to an embodiment of this application;
FIG. 5 is a schematic diagram of temperature calibration according to an embodiment of this application;
FIG. 6 is a schematic diagram of measurement based on an ideal sensor according to an embodiment of this application; and
FIG. 7 is a schematic diagram of an entire signal data processing process according to an embodiment of this application.
FIG. 8 is a first structural diagram of a multi-working-electrode system according to an embodiment of this application;
FIG. 9 is a second structural diagram of a multi-working-electrode system according to an embodiment of this application;
FIG. 10 is a preparation principle diagram of a multi-working-electrode system according to an embodiment of this application;
FIG. 11 is a third structural diagram of a multi-working-electrode system according to an embodiment of this application; and
FIG. 12 is a fourth structural diagram of a multi-working-electrode system according to an embodiment of this application.

List of components and reference numerals:
10: substrate; 11: first substrate portion; 12: second substrate portion; 13: first wire; 14: second wire; 15: first contact; 16: second contact; 21: first working electrode; 22: second working electrode; 23: reference electrode; 24: counter electrode; 31: first outer membrane; 32: second outer membrane; 33: glucose oxidase; 34: glucose dehydrogenase; 40: cutting line; and 50: membrane-wrapped container.

### DETAILED DESCRIPTION

An embodiment of this application provides a continuous glucose concentration monitoring method based on a multi-working-electrode system.

In order to make a person skilled in the art better understand the technical solutions of this application, the following clearly and completely describes the technical solutions in the embodiments of this application with reference to the accompanying drawings in the embodiments of this application. Apparently, the described embodiments are only some of the embodiments of this application rather than all of the embodiments. All other embodiments obtained by a person of ordinary skill in the art based on the embodiments of this application without creative efforts shall fall within the protection scope of this application.

Process steps of the method according to this embodiment of this application are as follows:
S101: Obtain, by using a first working electrode, a first current value used for representing a glucose concentration of a host, and obtain, by using a second working electrode, a second current value used for representing the glucose concentration of the host, where the first working electrode and the second working electrode are separately and independently disposed on a same substrate; and the first working electrode includes a first parameter, and the second working electrode includes a second parameter.

In this embodiment of this application, the first working electrode includes a first type of enzyme and a first type of membrane, and the second working electrode includes a second type of enzyme and a second type of membrane. Specifically, the first type of enzyme includes a glucose oxidase, the first type of membrane includes an outer oxygen permeation membrane, the second type of enzyme includes a glucose dehydrogenase, the second type of membrane is an outer oxygen isolation membrane, and an oxygen permeability of the outer oxygen permeation membrane is greater than that of the outer oxygen isolation membrane.

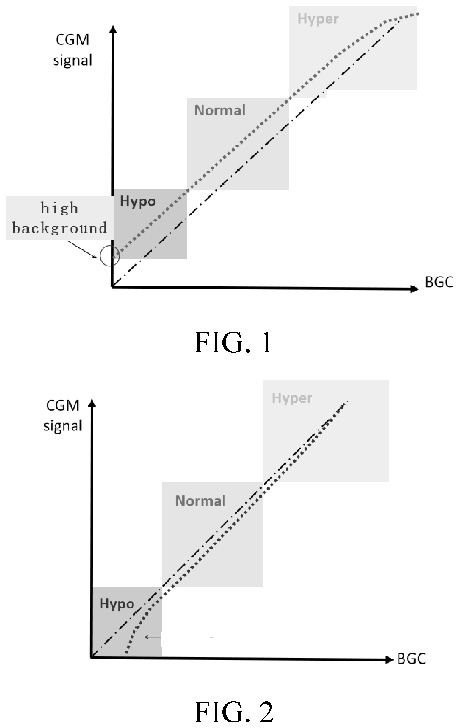

Refer to a schematic diagram of a measurement signal of a first working electrode shown in FIG. 1. A low blood glucose area (Hypo), a normal blood glucose area (Normal), and a high blood glucose area (Hyper) exist in FIG. 1. A horizontal coordinate BGC represents a current value, and a vertical coordinate CGM signal represents a blood glucose concentration. A working voltage corresponding to the first working electrode is relatively high, so that a background current (high background in FIG. 1) B is relatively large, and varies among persons. It is very difficult to effectively evaluate the background current B by using an in-vitro test. Consequently, test accuracy is affected. In addition, a part of H₂O₂ generated by a reaction between blood glucose and oxidase diffuses outward and cannot reach an electrode and be converted into current signal. Consequently, a calculated value of a blood glucose test is relatively low. This phenomenon is severer in a case of high blood glucose. In addition, in the case of high blood glucose, insufficient oxygen in the interstitial liquid limits a blood glucose oxidation reaction rate, attenuates a detection signal, and affects test accuracy of the first working electrode. Blood glucose refers to a glucose concentration in the blood.

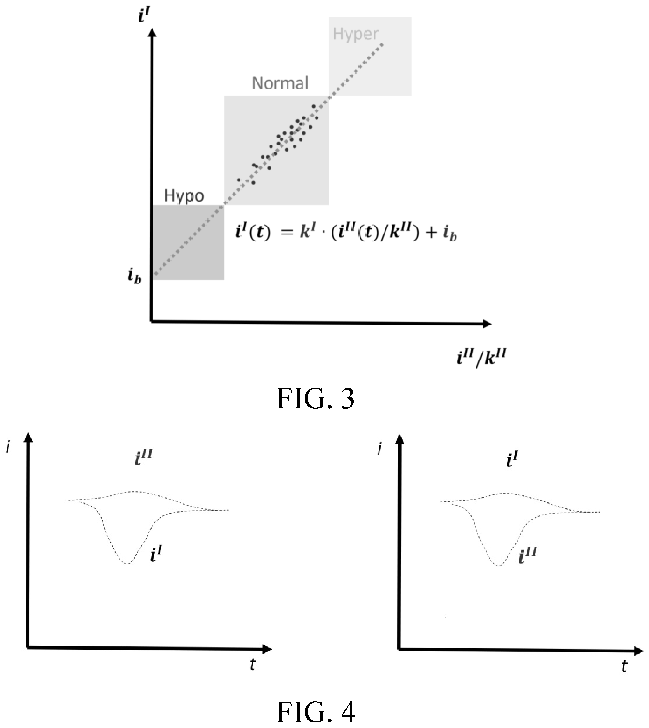

In this embodiment of this application, refer to a schematic diagram of a measurement signal of a second working electrode shown in FIG. 2. For specific content in FIG. 3, refer to the description of FIG. 1. The second working electrode corresponds to a low working voltage and a small background current, which are ignored. However, the measurement signal of the second working electrode is relatively low due to the oxygen effect, resulting in a relatively low measured blood glucose concentration, and when the blood glucose concentration is in a range of being normal and relatively low, the measurement accuracy of the second working electrode is relatively low. In addition, the second working electrode is relatively sensitive to a temperature, and the measurement signal is easily affected by a working temperature of the electrode.

It should be noted that, with regard to the foregoing S101, the following specific implementation solution is used:
One substrate is prepared for mounting two working electrodes. The first working electrode and the second working electrode are separately and independently disposed on the substrate. For the second parameter of the second working electrode, these parameters affect a response of the second working electrode to glucose, and the second parameter is obtained in advance and is a known parameter. For the first parameter of the first working electrode, a glucose concentration of the second working electrode is determined based on the second parameter and the second current value of the second working electrode, and the first parameter of the first working electrode is obtained based on the glucose concentration of the second working electrode and the first current value of the first working electrode.

S102: Determine a target glucose concentration according to the first current value as well as the first parameter and/or the second parameter as well as the second current value, where the first parameter is determined based on the second parameter and the second current value.

In S102 of this embodiment of this application, the glucose concentration corresponding to the first working electrode and the glucose concentration corresponding to the second working electrode are obtained through calculation according to the first current value and the second current value. The target glucose concentration is determined based on the glucose concentration of the first working electrode and the glucose concentration of the second working electrode.

Further, when the target glucose concentration is determined according to the first current value as well as the first parameter and/or the second parameter as well as the second current value, the first glucose concentration corresponding to the first working electrode is obtained based on the first current and the first parameter, the second glucose concentration corresponding to the second working electrode is obtained based on the second current value and the second parameter, and finally, the target glucose concentration is outputted based on the first glucose concentration and/or the second glucose concentration.

It should be noted that, the first glucose concentration is calculated according to a curve (for example, a relationship between a first current value and a concentration of a glucose solution) of the first working electrode by using the first current value and the first parameter. Similarly, the second glucose concentration is calculated according to a curve (for example, a relationship between a second current value and a concentration of the glucose solution) of the second working electrode by using the second current value and the second parameter. Then, a relationship between the first glucose concentration and the second glucose concentration is analyzed, and the target glucose concentration is outputted.

It should be noted that, through the foregoing content, the embodiments of this application have the following beneficial effects:
When the second glucose concentration is between the first preset value and the second preset value, both the first working electrode and the second working electrode can provide reliable measurement data, and the system flexibly selects the first glucose concentration or the second glucose concentration as the target glucose concentration.

Further, the first parameter is determined based on the second parameter and the second current value in the following specific implementation:
obtaining a second glucose concentration corresponding to the second working electrode based on the second current value and the second parameter when the glucose concentration of the host falls within a first threshold range; and obtaining the first parameter based on the glucose concentration corresponding to the second working electrode and the first current value. The first threshold range is a normal glucose concentration range, and the normal glucose concentration range is set according to an actual requirement. For example, the first threshold range is set to C₁-low to C₁-high mmol/L.

A value range of C₁-low is 3.9 to 7.8 mmol/L, and a value range of C₁-high is 6.1 to 10 mmol/L, and a value of C₁-low should be less than a value of C₁-high.

It should be noted that, through the foregoing content, the embodiments of this application have the following beneficial effects:
When the glucose concentrations fall within the first threshold range, the glucose concentration outputted by using the first working electrode and the glucose concentration outputted by using the second working electrode are substantially the same. Therefore, when the glucose concentration of the host falls within the first threshold range, the second glucose concentration corresponding to the second working electrode is obtained according to the second current value and the second parameter, and the first parameter of the first working electrode is calibrated according to the glucose concentration corresponding to the second working electrode and the first current value, thereby improving the accuracy of the measurement result of the first working electrode.

Further, the first parameter in this embodiment of this application includes a first sensitivity and a background current; and the second parameter includes a second sensitivity. With reference to FIG. 1 and FIG. 2, a relationship between a current value and a glucose concentration in the first working electrode and the second working electrode is a linear relationship. In a process of determining the first parameter, a plurality of first current values, a plurality of second current values, and a plurality of glucose concentration values corresponding to the second working electrode are set; When the first parameter is determined based on the second parameter, the first sensitivity and the background current are obtained based on the plurality of glucose concentrations corresponding to the second working electrode and the plurality of corresponding first current values.

A linear relationship of the first working electrode is Y=K₁X₁+B; and a linear relationship of the second working electrode is Y=K₂X₂, where Y is the measured glucose concentration, and K₁ is the first sensitivity, B is the background current, X₁ is the first current value, K₂ is the second sensitivity, and X₂ is the second current value. When the glucose concentration of the host falls within the first threshold range, measured glucose concentrations of the first working electrode and the second working electrode are the same at the same moment. A plurality of groups of data is measured at different moments. The plurality of groups of data includes glucose concentrations of the second working electrode at the plurality of moments and first current values at the plurality of moments. K₁ and B may be calculated. Calibration of the first parameter of the first working electrode is completed.

Further, the first parameter, such as the background current B and the sensitivity k^{I}, of the first working electrode is calibrated by using measurement data of the second working electrode within a normal glucose concentration range. In this way, a relationship between the glucose concentration of the in-vivo first working electrode in the body and the working current is obtained. Referring to a schematic diagram of calibration shown in FIG. 3, in FIG. 3, a vertical coordinate is a first current value *i*^{I}, and a horizontal coordinate is *i*^{II}/k^{II}. The calibration is performed in real time. Even if the first working electrode has a slow and long-term drift, the first working electrode is calculated and compensated for. In this way, the first working electrode does not need to be calibrated in advance, and only the second working electrode needs to be calibrated for production test.

It should be noted that, through the foregoing content, the embodiments of this application have the following beneficial effects:
The first sensitivity obtained through calibration more accurately reflects a degree of response of the first working electrode to a change in the glucose concentration, thereby improving the measurement sensitivity. In addition, because the background current of the first working electrode is relatively large and varies among persons, the background current is effectively subtracted from the measurement signal for the background current value in the first parameter obtained through calibration, thereby reducing a measurement error.

Further, the target glucose concentration is outputted based on the first glucose concentration and/or the second glucose concentration, and the first glucose concentration or the second glucose concentration is outputted as the target glucose concentration when the second glucose concentration is between a first preset value and a second preset value (a normal glucose concentration), where the first preset value is less than the second preset value. The first preset value and the second preset value are set according to an actual requirement. For example, the first preset value is set to C₂-low mmol/L, and the second preset value is set to C₂-high mmol/L.

A value range of C₂-low is 3.9 to 7.8 mmol/L, and a value range of C₂-high is 6.1 to 10 mmol/L, and a value of C₂-low should be less than a value of C₂-high.

It should be noted that, when the second glucose concentration is between the first preset value and the second preset value, both the first glucose concentration of the first working electrode and the second glucose concentration of the second working electrode may be considered as accurate glucose concentrations. Therefore, the first glucose concentration or the second glucose concentration is used as the target glucose concentration.

It should be noted that, through the foregoing content, the embodiments of this application have the following beneficial effects:
When the second glucose concentration is between the first preset value and the second preset value, both the first working electrode and the second working electrode can provide reliable measurement data, and the system flexibly selects the first glucose concentration or the second glucose concentration as the target glucose concentration.

Further, compared with that the second glucose concentration is between the first preset value and the second preset value, when the second glucose concentration is less than the first preset value, the first glucose concentration is outputted as the target glucose concentration, to avoid impact of the oxygen effect of the second working electrode.

It should be noted that, through the foregoing content, the embodiments of this application have the following beneficial effects:
When the second glucose concentration is less than the first preset value (a low glucose concentration), the second working electrode causes interference due to an oxygen effect. This interference can be avoided by using the data of the first working electrode as the target glucose concentration, thereby improving measurement accuracy.

Further, compared with that the second glucose concentration is between the first preset value and the second preset value, when the second glucose concentration is greater than the second preset value, a first weight value of the first working electrode and a second weight value of the second working electrode are determined based on the first parameter and the second parameter respectively; and the first glucose concentration and the second glucose concentration are converted into a target glucose concentration for output based on the first weight value and the second weight value.

It should be noted that, through the foregoing content, the embodiments of this application have the following beneficial effects:
When the second glucose concentration is greater than the second preset value (a high glucose concentration), the first working electrode is affected due to diffusion of hydrogen oxide, and the second working electrode is affected by the oxygen effect. Therefore, a measurement result is optimized according to a weight ratio of the two electrodes, to reduce a measurement error caused by a difference between characteristics of the electrodes.

Further, the first parameter includes a first sensitivity and a background current; the second parameter includes a second sensitivity; and a first weight value of the first working electrode and a second weight value of the second working electrode are determined based on the first parameter and the second parameter respectively, and the first weight value of the first working electrode and the second weight value of the second working electrode are separately determined based on a ratio of the first sensitivity to the second sensitivity, where a product of the first sensitivity and the first weight value is equal to a product of the second sensitivity and the second weight value.

It should be noted that, through the foregoing content, the embodiments of this application have the following beneficial effects:
By ensuring that the product of the first sensitivity and the first weight value is equal to the product of the second sensitivity and the second weight value, it is ensured that the weighted sensitivities of the two electrodes keep the same, thereby improving the accuracy of the measurement result.

Further, a glucose concentration needs to be measured in consideration of whether the interstitial liquid near the first working electrode and the second working electrode has sufficient oxygen, and is obtained by monitoring the difference between the first glucose concentration and the second glucose concentration and the difference between the first current value and the second current value when the interstitial liquid near the electrodes has insufficient oxygen. That is, in this embodiment of this application, before the target glucose concentration is outputted based on the first glucose concentration and/or the second glucose concentration, if the difference between the first glucose concentration and the second glucose concentration exceeds the third preset value and/or the difference between the first current value and the second current value exceeds the fourth preset value, it is determined that oxygen in the interstitial liquid near the electrodes is insufficient. In this case, the second glucose concentration is used as the target glucose concentration. The third preset value and the fourth preset value are set according to an actual situation.

It should be noted that, when the glucose concentration is low, measurement and reaction require relatively little oxygen, and the anoxia effect is relatively small. However, in a range of a normal glucose concentration and a high glucose concentration, anoxia attenuates a signal of the first working electrode, but has little impact on a signal of the second working electrode. If a schematic diagram of signal comparison shown in FIG. 4 appears, it is determined, at the left side of FIG. 4, that the interstitial liquid near the electrodes has insufficient oxygen, and in this case, the signal of the second working electrode needs to be used as a normal test signal, that is, the second glucose concentration is used as the target glucose concentration; and it is determined, at the right side of FIG. 4, that the interstitial liquid near the electrodes has excessively sufficient oxygen, and in this case, the signal of the first working electrode needs to be used as a normal test signal, that is, the first glucose concentration is used as the target glucose concentration.

It should be noted that, through the foregoing content, the embodiments of this application have the following beneficial effects:
If the difference between the first glucose concentration and the second glucose concentration exceeds the first preset value and/or the difference between the first current value and the second current value exceeds the second preset value, it is determined that oxygen in an interstitial liquid near an electrode is insufficient, and the insufficient oxygen causes incomplete reaction of the electrode, thereby affecting the current value. The second glucose concentration of the second working electrode is used as a target value, to reduce this error. In addition, in this embodiment of this application, working can continue in an environment with insufficient oxygen, thereby improving adaptability and reliability of the system.

Further, in this embodiment of this application, a case in which the first working electrode and the second working electrode fail needs to be further considered. If the change rate of the glucose concentration corresponding to the first working electrode and/or the second working electrode exceeds the fifth preset value, the corresponding working electrode is set to be failed. For example, the fifth preset value is set to 60%, the change rate of the glucose concentration corresponding to the first working electrode is set to 70%, and it is determined that the first working electrode is failed. Alternatively, if the change rate of the glucose concentration corresponding to the first working electrode and/or the second working electrode is less than the sixth preset value, the corresponding working electrode is set to be failed. The sixth preset value is less than the fifth preset value. For example, the sixth preset value is set to 5%, the change rate of the glucose concentration corresponding to the first working electrode is 1%, and it is determined that the first working electrode is failed. The fifth preset value and the sixth preset value are set according to an actual situation.

It should be noted that, through the foregoing content, the embodiments of this application have the following beneficial effects:
By monitoring the change rate of the glucose concentration, a working status of an electrode is determined in real time, thereby ensuring continuity and stability of a measurement process. In addition, a failed electrode causes an increase in a measurement error. The failed electrode is identified and excluded in advance, so that the measurement error is reduced, and accuracy of data is ensured.

Further, sensitivities of the first working electrode and the second working electrode to a temperature are different and have a large difference. Therefore, a temperature change is reflected in an electrode signal (glucose concentration). An algorithm is used to calculate a change in temperature according to a difference between two signals, and calibration and compensation are performed on the signals to remove impact of the temperature. For S102, when the target glucose concentration is outputted based on the first glucose concentration and/or the second glucose concentration, a first temperature coefficient of the first working electrode and a second temperature coefficient of the second working electrode are first obtained; a temperature difference is determined based on the first temperature coefficient, the second temperature coefficient, the first glucose concentration, and the second glucose concentration, where the temperature difference is a difference between a current actual measured temperature and a preset temperature; and the target glucose concentration is outputted based on the temperature difference, the first temperature coefficient, and the first glucose concentration; or the target glucose concentration is outputted based on the temperature difference, the second temperature coefficient, and the second glucose concentration. It should be noted that, the foregoing temperature refers to a human body temperature.

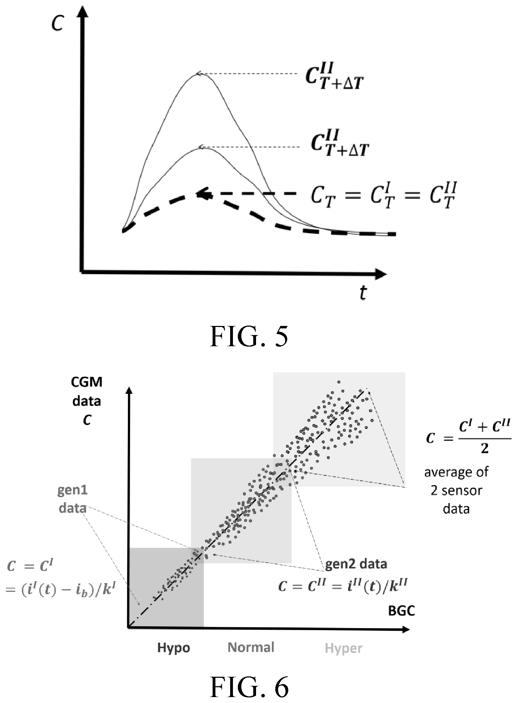

The temperature impact of the first working electrode and the second working electrode is expressed by using the following two equations: ${C}_{T + ΔT}^{I} = \left(1+{α}^{I}⋅ΔT\right) {C}_{T} ;$ and ${C}_{T + ΔT}^{II} = \left(1+{α}^{II}⋅ΔT\right) {C}_{T} ;$ where α^{I} is a temperature coefficient of the first working electrode, α^{II} is a temperature coefficient of the second working electrode, α^{I} and α^{II} are calibrated in advance in a laboratory, ${C}_{T + ΔT}^{I}$ is a current glucose concentration of the first working electrode, ${C}_{T + ΔT}^{II}$ is a current glucose concentration of the second working electrode, and C_{T} is an actual value of the glucose concentration obtained after calibration and compensation are performed on the first working electrode and the second working electrode. Therefore, C_{T} is the same in the first working electrode and the second working electrode. As a schematic diagram of temperature calibration shown in FIG. 5, when a temperature deviates from a normal temperature T, an actual temperature is T + ΔT, where ΔT is unknown, and the first working electrode and the second working electrode respectively give readings of the blood glucose concentration as ${C}_{T + ΔT}^{I}$ and ${C}_{T + ΔT}^{II} , {C}_{T} = {C}_{T}^{I} = {C}_{T}^{II} , { C}_{T}^{I}$ is an actual value of the glucose concentration obtained after calibration and compensation are performed on the first working electrode, and ${C}_{T}^{II}$ is an actual value of the glucose concentration obtained after calibration and compensation are performed on the second working electrode. By using the foregoing two formulas, it is learned that $\frac{{C}_{T + ΔT}^{I}}{{C}_{T + ΔT}^{II}} = \frac{1 + {α}^{I} ⋅ ΔT}{1 + {α}^{II} ⋅ ΔT}$. Therefore, ΔT is calculated, and then substituted into either of the foregoing two formulas, to calculate C_{T}, that is, the glucose concentration obtained by removing the temperature impact.

It should be noted that, through the foregoing content, the embodiments of this application have the following beneficial effects:
A temperature is one of important factors affecting a response of an electrode. By considering a temperature coefficient, the system can compensate for a blood glucose concentration measurement error caused by a temperature change, thereby improving accuracy of a measurement result. In addition, the temperature difference is determined and the temperature coefficient is applied, so that impact of the temperature on the measurement result is reduced, and the measurement accuracy of the blood glucose concentration is improved.

It should be noted that, to measure a more accurate glucose concentration, an ideal sensor is obtained by combining the first working electrode and the second working electrode. FIG. 6 is a schematic diagram of measurement based on an ideal sensor. In a low blood glucose concentration range (lower than a second threshold range), the first working electrode may be used, to avoid impact of a peroxide effect of the first working electrode, and C *= C' =* (*i'*(*t*) *- i_{b}*)/*k'* is used for a low blood glucose area (Hypo), is gen1 data in the figure, where C is a glucose concentration, *C'* is a glucose concentration of the first working electrode, *i*'(*t*) is a working current of the first working electrode, *i_{b}* is a background current of the first working electrode, and *k'* is a sensitivity coefficient of the first working electrode. In a normal blood glucose concentration range (within the second threshold range), both the first working electrode and the second working electrode may be used. A normal blood glucose area (Normal) is shown in the figure by using the second working electrode as an example. That is, an electrode whose C=C"="/ is shown in the figure as an example, that is,C = *C*" *= i"*(*t*)/*k"* is used and is gen2 data in the figure, where C" is a glucose concentration of the second working electrode, *i"*(*t*) is a working current of the second working electrode, and *k*" is a sensitivity coefficient of the second working electrode. In a high blood glucose concentration range (higher than the second threshold range), a combination of the first working electrode and the second working electrode may be used, and $C = \frac{C ′ + C "}{2}$ is used for a high blood glucose area (Hyper), and is average of 2 sensor data in the figure.

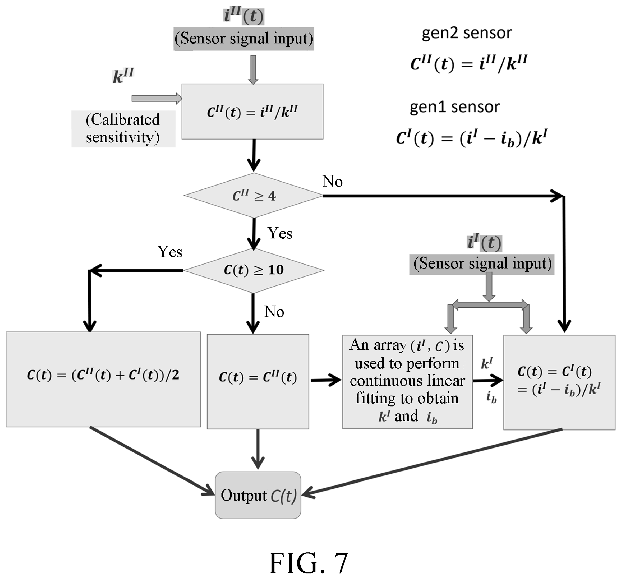

It should be noted that, with reference to the foregoing content, FIG. 7 is a schematic diagram of an entire signal data processing process according to an embodiment of this application. A signal input of a second working electrode in a sensor is *C"*(*t*) *= i"*(*t*)/*k".* In this case, a sensitivity coefficient *k"* of the second working electrode is calibrated, a signal input of a first working electrode is *C*(*t*) *= C'*(*t*) *=* (*i'*(*t*) *- i_{b}*)/*k'*, and whether the glucose concentration of the second working electrode is greater than C-low is determined. Whether the glucose concentration of the second working electrode is greater than or equal to C-high is determined if the glucose concentration is greater than or equal to C-low. If the glucose concentration is greater than or equal to C-high, $C \left(t\right) = \frac{C ′ \left(t\right) + C " \left(t\right)}{2}$ is used to measure the glucose concentration, and C(*t*) is outputted. If the glucose concentration is not greater than C-low, C(*t*) *= C'*(*t*) *=* (*i'*(*t*) *- i_{b}*)/*k'* is used to measure the glucose concentration, and C(*t*) is outputted. If the glucose concentration is greater than or equal to C-low and not greater than C-high, C(*t*) *= C'*(*t*) is used, C(*t*) is outputted, and an array (*i', C*) is used to perform continuous linear fitting to obtain *k'* and *i_{b}.*

The embodiments in this application are all described in a progressive manner. Refer to each other for the same or similar parts among the embodiments. Descriptions of each embodiment focus on differences from other embodiments.

A continuous glucose concentration monitoring apparatus provided in an embodiment of this application specifically includes the following modules:
a data obtaining module 901, configured to obtain, by using a first working electrode, a first current value used for representing a glucose concentration of a host, and obtain, by using a second working electrode, a second current value used for representing the glucose concentration of the host, where the first working electrode and the second working electrode are separately and independently disposed on a same substrate; and the first working electrode includes a first parameter, and the second working electrode includes a second parameter; and
a data processing module 902, configured to output a target glucose concentration according to the first current value, the first parameter, and the second current value, where the first parameter is determined based on the second parameter.

An embodiment of this application further provides a non-volatile computer storage medium, storing a computer executable instruction, and the computer executable instruction is configured to: perform the continuous glucose concentration monitoring method based on a multi-working-electrode system according to any one of the foregoing embodiments.

The embodiments in this application are all described in a progressive manner. Refer to each other for the same or similar parts among the embodiments. Descriptions of each embodiment focus on differences from other embodiments. Especially, device and medium embodiments are substantially similar to a method embodiment, and therefore is described briefly; for related parts, refer to partial descriptions in the method embodiment.

The device and the medium provided in the embodiments of this application are in a one-to-one correspondence with the method. Therefore, the device and the medium also have beneficial technical effects similar to those of the method corresponding to the device and the medium. The beneficial technical effects of the method are described in detail above, and therefore, the beneficial technical effects of the device and the medium are not described herein again.

A person skilled in the art needs to understand that the embodiments of the present application may be provided as a method, a system, or a computer program product. Therefore, this application may adopt a form of hardware-only embodiments, software-only embodiments, or embodiments combining software and hardware. Moreover, this application may adopt a form of a computer program product that is implemented on one or more computer-usable storage media (including but not limited to a disk memory, a CD-ROM, an optical memory, and the like) that include computer-usable program code.

The present application is described with reference to flowcharts and/or block diagrams of the method, the device (system), and the computer program product in the embodiments of the present application. It should be understood that computer program instructions may be used for implementing each procedure and/or block in the flowcharts and/or block diagrams and a combination of procedures and/or blocks in the flowcharts and/or block diagrams. These computer program instructions may be provided to a general-purpose computer, a special-purpose computer, an embedded processor, or a processor of another programmable data processing device to generate a machine, so that an apparatus configured to implement functions specified in one or more procedures in the flowcharts and/or one or more blocks in the block diagrams is generated by using instructions executed by a computer or a processor of another programmable data processing device.

These computer program instructions may alternatively be stored in a computer-readable storage device that can instruct a computer or another programmable data processing device to work in a specific manner, so that the instructions stored in the computer-readable storage device generate an artifact that includes an instruction apparatus. The instruction apparatus implements a specific function in one or more procedures in the flowcharts and/or in one or more blocks in the block diagrams.

These computer program instructions may alternatively be loaded onto a computer or another programmable data processing device, so that a series of operations and steps are performed on the computer or the another programmable device, thereby generating computer-implemented processing. Therefore, the instructions executed on the computer or the another programmable device provide steps for implementing a specific function in one or more processes in the flowcharts and/or in one or more blocks in the block diagrams.

In a typical configuration, a computing device includes one or more processors (CPUs), an input/output interface, a network interface, and a memory.

The memory may include a form such as a volatile memory, a random access memory (RAM), and/or a non-volatile memory such as a read-only memory (ROM) or a flash memory (flash RAM) in a computer-readable medium. The main memory is an example of the computer-readable medium.

The computer-readable medium includes a non-volatile medium and a volatile medium, a movable medium and a non-movable medium, which may implement storage of information by using any method or technology. The information may be computer-readable instructions, a data structure, a module of a program, or other data. Examples of a storage medium of a computer include, but are not limited to, a phase-change memory (PRAM), a static random access memory (SRAM), a dynamic random access memory (DRAM), a random access memory (RAM) of another type, a read-only memory (ROM), an electrically erasable programmable read-only memory (EEPROM), a flash memory or another memory technology, a compact disc read-only memory (CD-ROM), a digital versatile disc (DVD) or another optical storage, a cassette magnetic tape, tape and disk storage or another magnetic storage device or any other non-transmission medium that may be configured to store information that a computing device can access. According to the definitions in this specification, the computer-readable medium does not include transitory computer-readable media (transitory media), such as a modulated data signal and a carrier.

In an implementable embodiment of this application, a method for preparing a multi-working-electrode system for detecting a physiological parameter of a host is provided, and includes steps:
disposing a first working electrode 21 and a second working electrode 22 at an interval on a substrate 10, including:
the substrate 10 includes a first substrate portion 11 and a second substrate portion 12 that are disposed independently of each other, and the disposing a first working electrode 21 and a second working electrode 22 at an interval on a substrate 10 includes:
disposing the first working electrode 21 on the first substrate portion 11, and forming a first outer membrane 31 wrapping at least the first working electrode 21;
disposing the second working electrode 22 on the second substrate portion 12, and forming a second outer membrane 32 wrapping at least the second working electrode 22; and
combining the first substrate portion 11 and the second substrate portion 12 into the substrate 10.

Preferably, the first outer membrane 31 is an outer oxygen permeation membrane, the second outer membrane 32 is an outer oxygen isolation membrane, and an oxygen permeability of the outer oxygen permeation membrane is greater than an oxygen permeability of the outer oxygen isolation membrane.

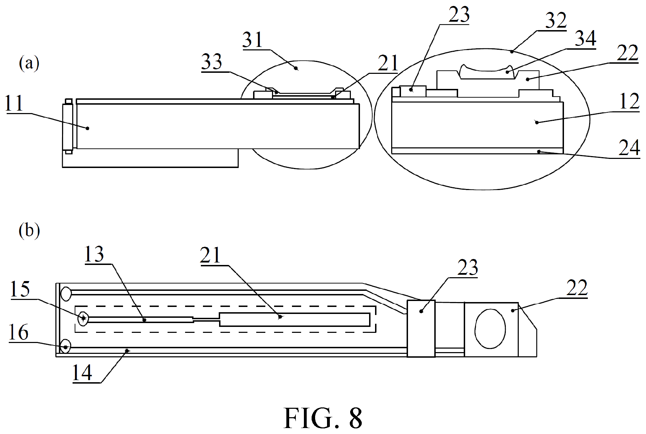

For the foregoing preparation method, refer to FIG. 8. (a) of FIG. 8 is a side view of the multi-working-electrode system, and (b) of FIG. 8 is a top view of the multi-working-electrode system. In this preparation manner, the first working electrode 21 undergoes an enzyme preparation process of a first-generation enzyme (glucose oxidase 33) on the first substrate portion 11, and then undergoes a wrapping process of the outer oxygen permeation membrane; and the second working electrode 22 undergoes an enzyme preparation process of a second-generation enzyme (glucose dehydrogenase 34) on the second substrate portion 12, and then undergoes a wrapping process of the outer oxygen isolation membrane. The two working electrodes are manufactured on base materials respectively and do not interfere with each other. In this way, respective enzyme and membrane processes of the working electrodes can be manufactured in respective optimized conditions, so as to obtain a multi-working-electrode system having optimal performance.

Referring to (b) of FIG. 8, in this setting, the first working electrode 21 may be embedded in a frame of the second working electrode 22. Referring to (b) of FIG. 8, a dashed line box is an embedding part of the first working electrode 21 and the second working electrode 22.

Specifically, each of the enzyme preparation process of the glucose oxidase 33 of the first working electrode 21, the wrapping process of the outer oxygen permeation membrane, the enzyme preparation process of the glucose dehydrogenase 34 of the second working electrode 22, and the wrapping process of the outer oxygen isolation membrane that are mentioned above uses an existing manner in the existing technology, and does not constitute a limitation on the multi-working-electrode system of this embodiment.

As a specific implementation, the preparation of the multi-working-electrode system further includes a reference electrode 23 and a counter electrode 24. The first working electrode 21 and the second working electrode 22 share one reference electrode 23 and one counter electrode 24, and each two electrodes are isolated from each other by using an insulation layer. Refer to an existing manner in the existing technology for a preparation method and positions of the reference electrode 23 and the counter electrode 24 in this embodiment, which does not cause interference to the multi-working-electrode system in this embodiment.

As an exemplary implementation, in this embodiment, the first working electrode 21 is a platinum electrode, the second working electrode 22 is a carbon electrode, the reference electrode 23 is a silver electrode or a silver chloride electrode, and the counter electrode 24 is a carbon electrode or a gold electrode.

In another implementable embodiment of this application, a method for preparing a multi-working-electrode system for detecting a physiological parameter of a host is provided, and includes steps:
disposing a first working electrode 21 and a second working electrode 22 on a same substrate 10 and at an interval in a longitudinal direction of the substrate 10.

Preferably, a first outer membrane 31 is an outer oxygen permeation membrane, a second outer membrane 32 is an outer oxygen isolation membrane, and an oxygen permeability of the first outer membrane 31 is greater than an oxygen permeability of the second outer membrane 32.

According to the preparation method, the first working electrode 21 and the second working electrode 22 are prepared on the same substrate 10, thereby simplifying an assembly process of Embodiment 1. The preparation method specifically includes the following three specific solutions:
The first solution and the second solution further include:
forming a first outer membrane 31 wrapping at least the first working electrode 21 on the substrate 10; and
forming a second outer membrane 32 wrapping at least the second working electrode 22 on the substrate 10, where the first outer membrane 31 and the second outer membrane 32 are disposed at an interval in the longitudinal direction of the substrate 10. Specifically, the first solution and the second solution are as follows:
First solution:

The substrate 10 includes a first part and a second part that are at least partially connected to each other, and the first part and the second part are bent in a direction away from each other to partially separate the two parts, where the first working electrode 21 is disposed at the first part, and the second working electrode 22 is disposed at the second part;
a first outer membrane 31 wrapping at least the first working electrode 21 is formed on the first part, and a second outer membrane 32 wrapping at least the second working electrode 22 is formed on the second part; and
the first part and the second part are restored to a non-separated state.

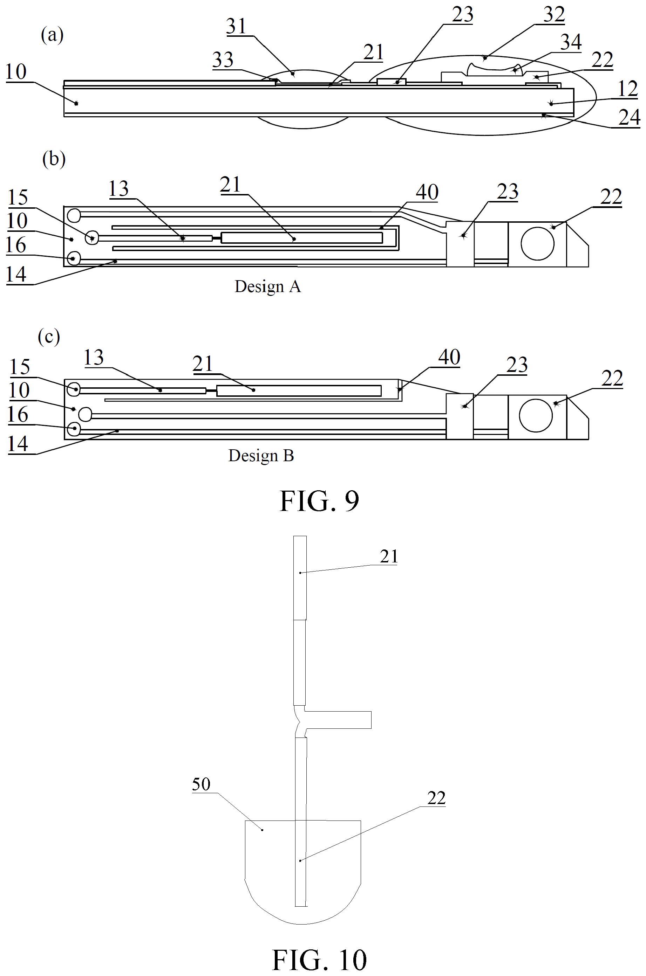

For the solution, refer to FIG. 9 and FIG. 10. (a) of FIG. 2 is a side view of a multi-working-electrode system in this solution. (b) of FIG. 9 is a top view of a multi-working-electrode system designed in the solution A. (c) of FIG. 9 is a top view of a multi-working-electrode system designed in the solution B. FIG. 10 is a preparation principle diagram of performing a membrane process on a first working electrode 21 and a second working electrode 22 in this solution.

Referring to (a) of FIG. 9, in the preparation solution, a first working electrode 21 and a second working electrode 22 are disposed on a same substrate 10. Referring to FIG. 10, a first part and a second part are bent in a direction away from each other to partially separate the two parts, and non-separated parts remain connected, to implement wrapping of the first outer membrane 31 and the second outer membrane 32. After the wrapping is completed, the first part and the second part are restored to the substrate 10 in the non-separated state.

Preferably, a first outer membrane 31 is an outer oxygen permeation membrane, a second outer membrane 32 is an outer oxygen isolation membrane, and an oxygen permeability of the first outer membrane 31 is greater than an oxygen permeability of the second outer membrane 32.

Referring to (b) of FIG. 9 and (c) of FIG. 9, before the substrate 10 is cut, this solution further includes:
forming a first wire 13 and a second wire 14 on the substrate 10, where the first wire 13 is connected to a first contact 15 and the first working electrode 21 on the substrate 10, the second wire 14 is connected to a second contact 16 and the second working electrode 22 on the substrate 10, and the first wire 13 and the second wire 14 are insulated from each other.

Same as Embodiment 1, the preparation of the multi-working-electrode system further includes a reference electrode 23 and a counter electrode 24. The first working electrode 21 and the second working electrode 22 share one reference electrode 23 and one counter electrode 24, and each two electrodes are isolated from each other by using an insulation layer. Refer to an existing manner in the existing technology for a preparation method and positions of the reference electrode 23 and the counter electrode 24 in this embodiment, which does not cause interference to the multi-working-electrode system in this embodiment.

As an exemplary implementation, in this embodiment, the first working electrode 21 is a platinum electrode, the second working electrode 22 is a carbon electrode, the reference electrode 23 is a silver electrode or a silver chloride electrode, and the counter electrode 24 is a carbon electrode or a gold electrode.

Refer to (b) of FIG. 9, which is a specific structure of the design A of the solution. In the structure, a first wire 13 connected to a first working electrode 21 is disposed between a reference wire connected to a reference electrode 23 and a second wire 14 connected to a second working electrode 22. In the design A, the first working electrode 21 is cut along a cutting line 40 (three sides of the first working electrode 21 are cut), so that the first working electrode 21 and the second working electrode 22 can be bent and separated from each other according to the structure of the design, and then preparation of an enzyme process and a membrane process is performed on the first working electrode 21 and the second working electrode 22. After the preparation is completed, the first part and the second part are restored to the substrate 10 in a non-separated state.

Refer to (c) of FIG. 9, which is a specific structure of the design B of the solution. In the structure, a first wire 13 connected to a first working electrode 21 is disposed on a side of a reference wire connected to a reference electrode 23 and far away from a second wire 14 connected to a second working electrode 22. In the design B, the first working electrode 21 is cut along a cutting line 40 (two sides of the first working electrode 21 are cut), so that the first working electrode 21 and the second working electrode 22 can be bent and separated from each other according to the structure of the design, and then preparation of an enzyme process and a membrane process is performed on the first working electrode 21 and the second working electrode 22. After the preparation is completed, the first part and the second part are restored to the substrate 10 in a non-separated state.

After the cutting is performed according to the design A and the design B that are described above, for the operations of performing the enzyme process and the membrane process on the first working electrode 21 and the second working electrode 22, refer to in FIG. 10. The multi-working-electrode system has soft needle areas separated but contact areas connected. During the enzyme process and the membrane coating process, soft needles of an electrode are bent by 90° in opposite directions and are fixed, and the enzyme process and the membrane coating process are separately performed on two opposite ends.

The above-mentioned enzyme process and membrane coating process are specifically as follows: The first working electrode 21 undergoes an enzyme preparation process of a first-generation enzyme (glucose oxidase 33), and then undergoes a wrapping process of the outer oxygen permeation membrane; and the second working electrode 22 undergoes an enzyme preparation process of a second-generation enzyme (glucose dehydrogenase 34), and then undergoes a wrapping process of the outer oxygen isolation membrane, where the wrapping of the outer oxygen permeation membrane and the wrapping of the outer oxygen isolation membrane are separately performed by using respective membrane wrapping containers 50.

Specifically, each of the enzyme preparation process of the glucose oxidase 33 of the first working electrode 21, the wrapping process of the outer oxygen permeation membrane, the enzyme preparation process of the glucose dehydrogenase 34 of the second working electrode 22, and the wrapping process of the outer oxygen isolation membrane that are mentioned above uses an existing manner in the existing technology, and does not constitute a limitation on the multi-working-electrode system of this embodiment.

### Second solution:

A first outer membrane 31 wrapping at least the first working electrode 21 and the second working electrode 22 is formed on the substrate 10; and
the first outer membrane 31 is covered with a second outer membrane 32 wrapping the second working electrode 22.

Preferably, a first outer membrane 31 is an outer oxygen permeation membrane, a second outer membrane 32 is an outer oxygen isolation membrane, and an oxygen permeability of the first outer membrane 31 is greater than an oxygen permeability of the second outer membrane 32.

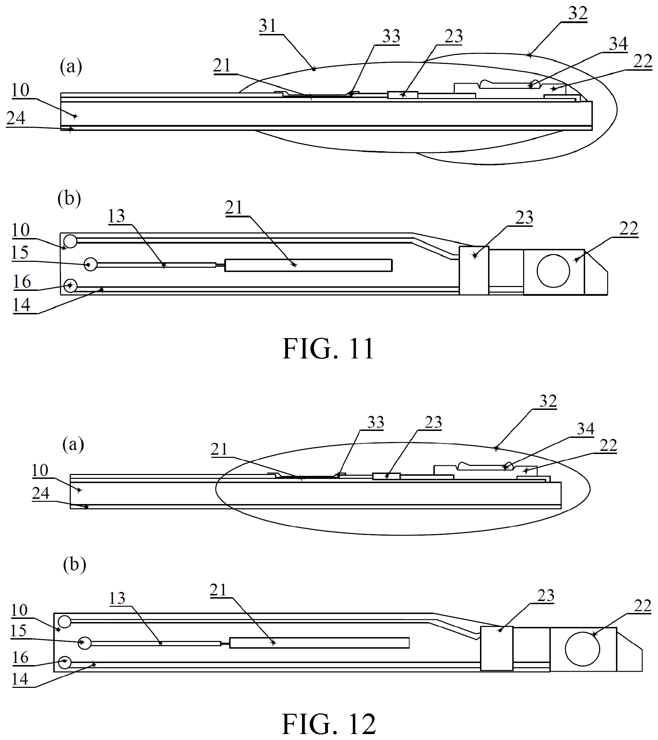

For this solution, refer to FIG. 11. (a) of FIG. 11 is a side view of the multi-working-electrode system in this solution, and (b) of FIG. 11 is a top view of the multi-working-electrode system in this solution. In this solution, the substrate 10 is not cut, the first working electrode 21 and the second working electrode 22 are both integral in the preparation of the enzyme process and the membrane process, and the two electrodes are on a same soft needle, so that the preparation process is simpler.

In this design solution, an electrode part of the second working electrode 22 is close to the tip of the soft needle. After the first working electrode 21 undergoes the enzyme preparation process of the first-generation enzyme (glucose oxidase 33) and the second working electrode 22 undergoes the enzyme preparation process of the second-generation enzyme (glucose dehydrogenase 34), the first working electrode 21 and the second working electrode 22 are coated with an outer oxygen permeation membrane. Both of the two electrodes are wrapped. Then, an electrode part of the second working electrode 22 close to the tip of the soft needle is coated with an outer oxygen isolation membrane. The second working electrode 22 needs to be isolated from oxygen, and therefore is first wrapped with the outer oxygen permeation membrane and then wrapped with the outer oxygen isolation membrane, and the outer oxygen isolation membrane can well isolate oxygen on an outer layer. Therefore, normal operation of the second working electrode 22 can be ensured.

Specifically, each of the enzyme preparation process of the glucose oxidase 33 of the first working electrode 21, and the enzyme preparation process of the glucose dehydrogenase 34 of the second working electrode 22, the wrapping process of the outer oxygen permeation membrane, and the wrapping process of the outer oxygen isolation membrane that are mentioned above uses an existing manner in the existing technology, and does not constitute a limitation on the multi-working-electrode system of this embodiment.

### Third solution:

A second outer membrane 32 wrapping at least the first working electrode 21 and the second working electrode 22 is formed on the substrate 10.

Preferably, the second outer membrane 32 is an outer oxygen isolation membrane.

For this solution, refer to FIG. 12. (a) of FIG. 12 is a side view of the multi-working-electrode system in this solution, and (b) of FIG. 12 is a top view of the multi-working-electrode system in this solution. The first working electrode 21 and the second working electrode 22 are both integral in the preparation of the enzyme process and the membrane process, and the two electrodes are on a same soft needle. In addition, the first working electrode 21 and the second working electrode 22 undergo only the wrapping of the outer oxygen isolation membrane, but do not undergo the wrapping of the outer oxygen permeation membrane. Compared with the second solution, a preparation process is simpler.

In this design solution, after the first working electrode 21 undergoes the enzyme preparation process of the first-generation enzyme (glucose oxidase 33) and the second working electrode 22 undergoes the enzyme preparation process of the second-generation enzyme (glucose dehydrogenase 34), the first working electrode 21 and the second working electrode 22 are coated with an outer oxygen isolation membrane. Both of the two electrodes are covered. In this disposition manner, although the first working electrode 21 is wrapped with the outer oxygen isolation membrane, because the first working electrode 21 can work with a low blood glucose concentration and needs very little oxygen, a small amount of oxygen can penetrate to the first working electrode 21 for reaction through the outer oxygen isolation membrane, and the measurement effect of the first working electrode 21 is not affected. In addition, the measurement effect of the second working electrode 22 can be ensured by disposing the outer oxygen isolation membrane.

In the three solutions of this embodiment, the multi-working-electrode system includes a reference electrode 23 and a counter electrode 24. The first working electrode 21 and the second working electrode 22 share one reference electrode 23 and one counter electrode 24, and each two electrodes are isolated from each other by using an insulation layer. Refer to an existing manner in the existing technology for a preparation method and positions of the reference electrode 23 and the counter electrode 24 in this embodiment, which does not cause interference to the multi-working-electrode system in this embodiment.

As an exemplary implementation, in this embodiment, the first working electrode 21 is a platinum electrode, the second working electrode 22 is a carbon electrode, the reference electrode 23 is a silver electrode or a silver chloride electrode, and the counter electrode 24 is a carbon electrode or a gold electrode.

In an implementable embodiment of this application, a multi-working-electrode system for detecting a glucose concentration is provided, and includes:
a substrate 10;
a first working electrode 21, including a glucose oxidase 33; and
a second working electrode 22, including a glucose dehydrogenase 34, where
the first working electrode 21 and the second working electrode 22 are disposed at an interval on the substrate 10, independently generate a first current value and a second current value used for representing a glucose concentration of the host respectively, and determine the glucose concentration of the host according to the first current value and the second current value; and
the first working electrode 21 is wrapped with a first outer membrane 31, the second working electrode 22 is wrapped with a second outer membrane 32, and an oxygen permeability of the first outer membrane 31 is greater than an oxygen permeability of the second outer membrane 32.

The multi-working-electrode system is prepared by using the preparation method in the foregoing embodiment. A specific structure and a preparation method are not described in detail. A method for detecting a glucose concentration online by the multi-working-electrode system is described in detail in this embodiment, and details are as follows:
Process steps of the method according to this embodiment of this application are as follows:
S101: Obtain, by using a first working electrode 21, a first current value that can represent a glucose concentration of a host, and obtain, by using a second working electrode 22, a second current value that can represent the glucose concentration of the host, where the first working electrode 21 and the second working electrode 22 are separately and independently disposed on a same substrate 10, and a first parameter of the first working electrode 21 is determined based on a second parameter of the second working electrode 22.

In this embodiment of this application, the first working electrode 21 includes the glucose oxidase 33. A working voltage corresponding to the first working electrode is relatively high, so that a background current is relatively large, and varies among persons. It is very difficult to effectively evaluate the background current B by using an in-vitro test. Consequently, test accuracy is affected. In addition, a part of H₂O₂ generated by a reaction between blood glucose and oxidase diffuses outward and cannot reach an electrode and be converted into current signal. Consequently, a calculated value of a blood glucose test is relatively low. This phenomenon is severer in a case of high blood glucose. In addition, in the case of high blood glucose, insufficient oxygen in the interstitial liquid limits a blood glucose oxidation reaction rate, attenuates a detection signal, and affects test accuracy of the first working electrode. Blood glucose refers to a glucose concentration in the blood.

In this embodiment of this application, the second working electrode 22 includes the glucose dehydrogenase 34. The second working electrode corresponds to a low working voltage and a small background current, which are ignored. However, the measurement signal of the second working electrode is relatively low due to the oxygen effect, resulting in a relatively low measured blood glucose concentration, and when the blood glucose concentration is in a range of being normal and relatively low, the measurement accuracy of the second working electrode is relatively low. In addition, the second working electrode is relatively sensitive to a temperature, and the measurement signal is easily affected by a working temperature of the electrode.

Therefore, in different blood glucose conditions, by combining the first working electrode and the second working electrode, a more accurate glucose concentration can be measured. In a low blood glucose concentration range (lower than C₁-low mmol/L), the first working electrode may be used, to avoid impact of a peroxide effect of the second working electrode. In a normal blood glucose concentration range (from C₁-low mmol/L to C₁-high mmol/L), either of the first working electrode and the second working electrode may be used. In a high blood glucose concentration range (higher than C₁-high mmol/L), a combination of the first working electrode and the second working electrode may be used, and an average value of blood glucose concentrations measured by the two electrodes is taken as a final blood glucose concentration.

A value range of C₁-low is 3.9 to 7.8 mmol/L, and a value range of C₁-high is 6.1 to 10 mmol/L, and a value of C₁-low should be less than a value of C₁-high.

It should be noted that, with regard to the foregoing S101, the following specific implementation solution may be used:
Electrode mounting: One substrate 10 is prepared for mounting two working electrodes. A first working electrode 21 and a second working electrode 22 are separately and independently disposed on the substrate 10, to ensure that there is a sufficient distance between the two electrodes to avoid mutual interference.

Electrode parameter setting: The second parameter of the second working electrode 22 may be a sensitivity coefficient of the second working electrode 22, these parameters affect a response of the second working electrode 22 to glucose, and the second parameter may be obtained in advance and is a known parameter. For the first parameter of the first working electrode 21, a glucose concentration of the second working electrode 22 may be determined based on the second parameter and the second current value of the second working electrode 22, and the first parameter of the first working electrode 21 is obtained based on the glucose concentration of the second working electrode 22 and the first current value of the first working electrode 21.

Current measurement: Current measurement is performed on the two working electrodes by using an appropriate measurement device. The first current value representing a glucose concentration of a host is obtained by using the first working electrode 21. Similarly, the second current value representing the glucose concentration of the host is obtained by using the second working electrode 22.

S102: Output a target glucose concentration according to the first current value and the second current value.

In S102 of this embodiment of this application, the glucose concentration corresponding to the first working electrode 21 and the glucose concentration corresponding to the second working electrode 22 may be respectively determined according to the first current value and the second current value. The glucose concentration of the host may be determined based on the glucose concentration of the first working electrode 21 and the glucose concentration of the second working electrode 22. A specific determining manner of the target glucose concentration may be a manner based on, for example, an average value or a weight value.

Further, when the glucose concentration of the host is outputted according to the first current value and the second current value, a first glucose concentration corresponding to the first working electrode 21 may be first obtained based on the first current and the first parameter; a second glucose concentration corresponding to the second working electrode 22 is then obtained based on the second current value and the second parameter; and the target glucose concentration is finally outputted based on the first glucose concentration and/or the second glucose concentration.

It should be noted that, the first glucose concentration may be calculated according to a curve (for example, a relationship between a current value and a concentration of a glucose solution) of the first working electrode 21 by using the first current value and the first parameter. Similarly, the second glucose concentration is calculated according to a curve of the second working electrode 22 by using the second current value and the second parameter. Then, a relationship between the first glucose concentration and the second glucose concentration is analyzed, a difference between the first glucose concentration and the second glucose concentration may be considered, and the target glucose concentration is outputted based on the difference between the first glucose concentration and the second glucose concentration.

It should be noted that, through the foregoing content, the embodiments of this application have the following beneficial effects:
Improvement in measurement precision: An actual glucose concentration can be reflected more precisely by separately calculating the glucose concentrations corresponding to the first working electrode and the second working electrode. Such a dual-parameter measurement method helps to reduce an error in single measurement, thereby improving overall measurement precision.

Enhancement in anti-interference capability: Because each electrode may respond to different interference factors differently, environmental interference and cross-sensitivity can be identified and eliminated more effectively by comparing measurement results of two electrode, thereby improving reliability of a detection result.

Cross-validation: By outputting the glucose concentrations corresponding to the first working electrode and the second working electrode, cross-validation may be performed. If the measurement results of the two electrodes are consistent, the confidence of the measurement results may be enhanced. If the results are inconsistent, the reason may be further analyzed, thereby improving robustness of the detection system.

Further, based on characteristics of the first working electrode and the second working electrode, in this embodiment of this application, the first parameter of the first working electrode needs to be calibrated based on the second parameter of the second working electrode. A specific calibration process is as follows: The first current value that can represent a glucose concentration of a host is obtained by using the first working electrode; the second current value that can represent the glucose concentration of the host is obtained by using the second working electrode; a glucose concentration corresponding to the second working electrode is obtained based on the second current value and the second parameter; and when the glucose concentration corresponding to the second working electrode falls within the first threshold range, the first parameter may be obtained based on the glucose concentration and the first current value. The first threshold range may be a normal glucose concentration range, and the normal glucose concentration range may be set according to an actual requirement. For example, the first threshold range is set to C₂-low to C₂-high mmol/L.

A value range of C₂-low is 3.9 to 7.8 mmol/L, and a value range of C₂-high is 6.1 to 10 mmol/L, and a value of C₂-low should be less than a value of C₂-high.

It should be noted that, the first parameter is a background current and a sensitivity of the first working electrode, and the second parameter is a sensitivity of the second working electrode.

It should be noted that, through the foregoing content, the embodiments of this application have the following beneficial effects:
Calibrating the first working electrode by using the second working electrode can ensure that a precise calibration process is performed on the first working electrode before measurement, thereby improving accuracy of a measurement result.

It should be further noted that, the terms "include", "comprise", or any variants thereof are intended to cover a non-exclusive inclusion. Therefore, a process, method, article, or device that includes a series of elements not only includes such elements, but also includes other elements not specified expressly, or may include inherent elements of the process, method, article, or device. Unless otherwise specified, an element limited by "including a/an..." does not exclude that other same elements exist in the process, method, article, or device including the element.

The foregoing embodiments are merely intended for describing the technical solutions of this application, but not for limiting this application. Although this application is described in detail with reference to the foregoing embodiments, it should be understood that a person of ordinary skill in the art still makes modifications to the technical schemes described in the foregoing embodiments or makes equivalent replacements to some technical features thereof. However, the modifications or replacements do not make the essence of the corresponding technical solutions depart from the spirit and scope of the technical solutions of embodiments of this application, and shall fall within the protection scope of this application.

## Claims

1. A continuous glucose concentration monitoring method based on a multi-working-electrode system, comprising:
obtaining, by using a first working electrode, a first current value used for representing a glucose concentration of a host, and obtaining, by using a second working electrode, a second current value used for representing the glucose concentration of the host, wherein the first working electrode and the second working electrode are separately and independently disposed on a same substrate; and the first working electrode has a first parameter, and the second working electrode has a second parameter; and
outputting a target glucose concentration according to the first current value as well as the first parameter and/or the second parameter as well as the second current value,
wherein the first parameter is determined based on the second parameter and the second current value.

2. The method according to claim 1, wherein the outputting a target glucose concentration according to the first current value as well as the first parameter and/or the second parameter as well as the second current value comprises:
obtaining a first glucose concentration corresponding to the first working electrode based on the first current and the first parameter;
obtaining a second glucose concentration corresponding to the second working electrode based on the second current value and the second parameter; and
outputting the target glucose concentration based on the first glucose concentration and/or the second glucose concentration.

3. The method according to claim 1 or claim 2, wherein the determining the first parameter based on the second parameter and the second current value comprises:
obtaining a second glucose concentration corresponding to the second working electrode based on the second current value and the second parameter;
when the second glucose concentration of the host falls within a first threshold range, obtaining the first parameter based on the second glucose concentration and the first current value.

4. The method according to claim 2 or claim 3, wherein the outputting the target glucose concentration based on the first glucose concentration and/or the second glucose concentration comprises:
setting a first preset value and a second preset value, wherein the first preset value is less than the second preset value,
outputting the first glucose concentration or the second glucose concentration as the target glucose concentration when the second glucose concentration is between the first preset value and the second preset value,
outputting the first glucose concentration as the target glucose concentration when the second glucose concentration is less than the first preset value,
when the second glucose concentration is greater than the second preset value, the method further comprises
determining a first weight value of the first working electrode and a second weight value of the second working electrode based on the first parameter and the second parameter respectively; and
determining the target glucose concentration based on the first weight value, the second weight value, the first glucose concentration, and the second glucose concentration; optionally, the first parameter comprises a first sensitivity and a background current; the second parameter comprises a second sensitivity, wherein
determining the first weight value and the second weight value comprises:
separately determining the first weight value of the first working electrode and the second weight value of the second working electrode based on a ratio of the first sensitivity to the second sensitivity, wherein a product of the first sensitivity and the first weight value is equal to a product of the second sensitivity and the second weight value.

5. The method according to claim 2 or claim 3, further comprising:
setting, if a change rate of the glucose concentration corresponding to the first working electrode and/or the second working electrode exceeds a fifth preset value, the corresponding working electrode to be failed; or
setting, if a change rate of the glucose concentration corresponding to the first working electrode and/or the second working electrode is less than a sixth preset value, the corresponding working electrode to be failed, wherein the sixth preset value is less than the fifth preset value.

6. The method according to claim 2 or claim 3, wherein the outputting the target glucose concentration based on the first glucose concentration and/or the second glucose concentration comprises:
obtaining a first temperature coefficient of the first working electrode and a second temperature coefficient of the second working electrode;
determining a temperature difference based on the first temperature coefficient, the second temperature coefficient, the first glucose concentration, and the second glucose concentration, wherein the temperature difference is a difference between a current actual measured temperature and a preset temperature; and
outputting the target glucose concentration based on the temperature difference, the first temperature coefficient, and the first glucose concentration; or
outputting the target glucose concentration based on the temperature difference, the second temperature coefficient, and the second glucose concentration.

7. A continuous glucose concentration monitoring device based on a multi-working-electrode system, comprising:
a substrate,
a first working electrode, wherein the first working electrode has a first parameter,
a second working electrode, wherein the second working electrode has a second parameter;
wherein the first working electrode and the second working electrode are independently disposed on the substrate,
and a processing unit configured to perform the steps of the method according to any one of claims 1-6.

8. A computer program comprising instructions which, when the program is executed by a computer, cause the device of claim 7 to carry out the steps of method of any one of claim 1 - 6.

9. A computer-readable medium having stored thereon the computer program of claim 8.

10. A method for preparing continuous glucose concentration monitoring device based on a multi-working-electrode system, comprising:
disposing a first working electrode and a second working electrode at an interval on a substrate, wherein
the first working electrode is configured to independently generate a first current value used for representing a first physiological parameter, the second working electrode is configured to independently generate a second current value used for representing a second physiological parameter,
an output glucose concentration of the host is obtained based on the first current value and the second current value,
wherein at least one of the first physiological parameter and the second physiological parameter is a glucose concentration, optionally the first physiological parameter and the second physiological parameter are both glucose concentrations, the first working electrode comprises a glucose oxidase, and the second working electrode comprises a glucose dehydrogenase.

11. A method according to claim 10, wherein the first working electrode comprises a first outer membrane wrapping at least the first working electrode and the second working electrode comprises a second outer membrane wrapping at least the second working electrode; and an oxygen permeability of the first outer membrane is greater than an oxygen permeability of the second outer membrane.

12. The method according to claim 11, wherein the substrate comprises a first substrate portion and a second substrate portion that are disposed independently of each other, and the disposing a first working electrode and a second working electrode at an interval on a substrate comprises:
disposing the first working electrode on the first substrate portion, and forming the first outer membrane wrapping at least the first working electrode;
disposing the second working electrode on the second substrate portion, and forming the second outer membrane wrapping at least the second working electrode;, and
combining the first substrate portion and the second substrate portion into the substrate.

13. The method according to claim 11, wherein the first working electrode and the second working electrode are disposed on the same substrate and are disposed at an interval in a longitudinal direction of the substrate, and the method further comprises:
forming the first outer membrane wrapping at least the first working electrode on the substrate; and
forming the second outer membrane wrapping at least the second working electrode on the substrate, wherein the first outer membrane and the second outer membrane are disposed at an interval in the longitudinal direction of the substrate.

14. The method according to claim 11, wherein
the substrate comprises a first part and a second part that are at least partially connected to each other,
the method further comprises:
bending the first part and the second part in a direction away from each other to partially separate the two parts, wherein the first working electrode is disposed at the first part, and the second working electrode is disposed at the second part;
forming the first outer membrane wrapping at least the first working electrode on the first part, and forming the second outer membrane wrapping at least the second working electrode on the second part; and
restoring the first part and the second part to a non-separated state.

15. The method according to claim 11, further comprising:
forming a first wire and a second wire on the substrate, wherein the first wire is connected to a first contact and the first working electrode on the substrate, the second wire is connected to a second contact and the second working electrode on the substrate, and the first wire and the second wire are insulated from each other.
